# EUROPEAN PATENT SPECIFICATION

(11) **EP 2 739 605 B1**
(45) Date of publication and mention of the grant of the patent: **27.04.2016**
(21) Application number: 11770507.9
(22) Date of filing: 03.08.2011
(51) Int. Cl.: C07C 231/00, C07C 233/18

(54) **PROCESS FOR THE PREPARATION OF CRYSTALLINE FORM I OF AGOMELATINE**
VERFAHREN ZUR HERSTELLUNG EINER KRISTALLINEN FORM I VON AGOMELATIN
PROCÉDÉ DE PRÉPARATION DE LA FORME CRISTALLINE I DE L'AGOMÉLATINE

(43) Date of publication of application: 11.06.2014
(73) Proprietor: Laboratorio Chimico Internazionale S.p.A., 20121 Milano (IT)
(72) Inventor: BERTOLINI, Giorgio, 20090 Segrate (Ml) (IT); DE ANGELIS, Bruno, 20090 Segrate (Ml) (IT)
(74) Representative: Trupiano, Federica
(86) International application number: PCT/IB2011/001805
(87) International publication number: WO 2013/017903

(56) References cited:
- EP-A1- 2 319 827
- TINANT B ET AL: "N-Ä2-(7-METHOXY-1-NAPHTHYL)ETHYLÜACETAMID E, A POTENT MELATONIN ANALOG", ACTA CRYSTALLOGRAPHICA SECTION C. CRYSTAL STRUCTURE COMMUNICATIONS, MUNKSGAARD, COPENHAGEN, DK, vol. C50, no. 6, 1 January 1994 (1994-01-01), pages 907-910, XP009047983, ISSN: 0108-2701, DOI: 10.1107/S0108270193012922 cited in the application
- Petit S. et al.: "The Amorphous State" In: Hilfiker R. et al.: "Polymorphism in the Pharmaceutical Industry", 2006, WILEY-VCH, Weinheim, XP002666940, ISBN: 3-527-31146-7 pages 263-265, page 265, paragraph 10.3.3

## Description

### Summary of the invention

The present invention concerns a new process for the preparation of crystalline form I of agomelatine, in particular a freeze-drying process.

### Technical background

Agomelatine is an antidepressant drug having the following formula (I)

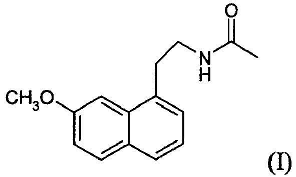

Agomelatine has been described and claimed in the patent EP0447285. In said document, agomelatine is obtained by reaction between 2-(7-methoxynaphth-1-yl)-ethylamine and acetyl chloride and classic recrystallisation from isopropyl ether. The agomelatine thus obtained has a melting point of 109-110°C; no information is provided on the type of crystalline form of the molecule.

Acta Cryst., 1994, C50, 907-910, reports for the first time a crystallographic analysis of agomelatine.

EP1564202 claims a form defined as "form II" which is obtained by reaction between 2-(7-methoxynaphth-1-yl)-ethylamine and sodium acetate and acetic anhydride in ethanol and washing of the precipitate with a water/ethanol mixture. Said crystalline form has a melting point of 108°C and presents specific crystallographic characteristics.

A series of successive patents describe further crystalline forms of agomelatine, in particular forms III to VI, prepared according to particular methods (crushing, atomization, crystallization in particular solvents, etc.).

EP2319827 describes a process for the preparation of crystalline form I of agomelatine, corresponding to the one described in Acta Cryst., 1994, C50, 907-910, which entails dissolving the agomelatine in an organic solvent miscible with water and pouring the solution thus obtained into water having a temperature equal to or below 30°C.

Also CN101704763A describes a process for the preparation of form I of agomelatine which is very similar to that of EP2319827, at a temperature of between 0°C and 100°C.

However, the processes described in EP2319827 and CN101704763A have some drawbacks. The applicant has tried to repeat said processes and has observed that they are not reproducible with constant results, because they produce different crystalline forms and often mixtures of different crystalline forms of agomelatine.

Hence there is a great need to find a process for the preparation of crystalline form I of agomelatine which overcomes the drawbacks of the prior art and can be constantly reproduced.

### Description of the invention

It has now surprisingly been found that it is possible to prepare crystalline form I of agomelatine, in a simple reliable way, by means of a freeze-drying process.

Thus, according to one of its embodiments, the invention concerns a process for the preparation of crystalline form I of agomelatine, which comprises freeze-drying a solution of agomelatine in an organic solvent.

By "crystalline form I of agomelatine", here otherwise called "polymorph I of agomelatine" or "agomelatine form I", we indicate the crystalline form described in Acta Cryst., 1994, C50, 907-910.

By "solution of agomelatine in an organic solvent" we indicate a solution prepared by dissolving the agomelatine in an organic solvent, cold or by heating, for example by heating to 30-40°C.

The starting agomelatine can have any crystalline form and be obtained according to any synthetic process.

By "organic solvent" we mean an organic solvent suitable for freeze-drying, able to dissolve the agomelatine and from which the agomelatine does not precipitate during the freeze-drying.

The organic solvent is chosen from C1-C6alcohols, for example tert-butanol and tert-amyl alcohol; preferred solvent is tert-butanol.

If desired or necessary, mixtures of organic solvents can be used, for example mixtures of the above-mentioned solvents.

The agomelatine/solvent ratio in the solution is not critical and depends on the solvent used, advantageously 10-100 g of agomelatine per litre of solvent can be dissolved, for example approximately 30-70 g, more preferably approximately 40-50 g of agomelatine per litre of solvent.

The term "freeze-drying" (or "liophylisation") here indicates the technological process which entails sublimation of the solvent by a solution previously frozen, at low temperature and under high vacuum. Freeze-drying is a method known in the pharmaceutical field and can be performed according to the conventional procedures, known to a person skilled in the art.

According to one embodiment of the invention, the solution of agomelatine in an appropriate solvent is frozen rapidly to approximately -20°C, or in any case at least to the freezing point of the solution used, in a suitable vessel. According to a preferred embodiment, the solution is kept frozen for a certain period, such as 24-60 hours, for example 48 hours.

Vacuum is applied to the frozen solution, for example a vacuum degree below 0.1 mbar, advantageously below or equal to 0.08 mbar, thus causing sublimation of the solvent.

The freeze-drying completion time depends, as is known, on the operating conditions used, such as the temperature, the degree of vacuum applied, the quantity of solvent to be eliminated, etc. A person skilled in the art is in any case able to choose the operating conditions and determine the time necessary for completion of the freeze-drying process.

According to one embodiment, the solution of agomelatine is if necessary filtered, rapidly frozen to an appropriate temperature (i.e. at least to the freezing temperature of the solution which obviously depends on the solvent used) and is placed in a freeze-dryer. The vacuum is then applied, for example equal to or below 0.08 mbar, to initiate the solvent sublimation process. At the end of the sublimation process the agomelatine is obtained in crystalline form I, in solid form.

Freeze-dryer equipments are also known in the art in which it is also possible to perform the freezing phase; in this case, all the freeze-drying, i.e. both the freezing stage and the solvent sublimation stages, are performed in said freeze-dryers.

The advantages of the process of the invention with respect to the prior art are evident. The freeze-drying process according to the invention always allows, in an entirely unexpected and unpredictable way, form I of agomelatine to be obtained, and represents a considerable progress with respect to the processes of crystallisation of the prior art which produce variable results that cannot be constantly reproduced.

Furthermore, with respect to the processes of the prior art cited above, which use a mixture of organic solvent and water, the possibility of using one single solvent allows recovery and recycling of said solvent more easily than the recycling of combinations of solvents miscible with one another, such as those used in the prior art.

However, the process of the invention does not preclude the use of mixtures of solvents, if desired or necessary.

According to another of its aspects, the invention concerns a pharmaceutical composition which comprises, as active ingredient, crystalline form I of agomelatine obtained according to the process of the invention.

The invention will now be better described, by way of non-limiting illustration, by means of the following experimental examples.

Experimental examples that demonstrate the drawbacks of the known art are also provided for comparative purposes.

### Experimental section

### Example 1

### Preparation of crystalline form I of agomelatine

3 g of agomelatine are dissolved in 70 ml of tert-butanol at 30-35°C. The solution is filtered, rapidly frozen to -20°C and left at this temperature for 48 hours. The solvent of the frozen solution is sublimated in a TELSTAR LYOALFA6 freeze-dryer under the following conditions: condenser temperature -50°C; vacuum degree 0.07 mbar; freeze-drying duration: 24 hours.

Agomelatine in crystalline form I is thus obtained, with quantitative yield.

### Example 2

### Preparation of crystalline form I of agomelatine

3 g of agomelatine are dissolved in 140 ml of tert-amyl alcohol at 30-35°C. The solution is filtered, rapidly frozen to -20°C and left at this temperature for 48 hours. The solvent of the frozen solution is sublimated in a TELSTAR LYOALFA6 freeze-dryer under the following conditions: condenser temperature -50°C; vacuum degree 0.07 mbar; freeze-drying duration: 48 hours.

Agomelatine in crystalline form I is thus obtained, with quantitative yield.

### Comparative examples

Examples of repetitions of the methods described in the prior art relative to the preparation of form I are given below.

### Comparative example (a)

10g of agomelatine are dissolved in 30 ml of methanol. The solution is added dropwise to 200 ml of water at a temperature below 30°C. The crystal formed is isolated by filtering and dried in a vacuum at 30°C. A mixture of the polymorphs I and II of agomelatine is obtained.

### Comparative example (b)

10g of agomelatine are dissolved in 50 ml of acetone. The solution is added dropwise to 1000 ml of water at a temperature below 30°C. The crystal formed is isolated by filtering and dried in a vacuum at 30°C. Polymorph II of agomelatine is obtained.

## Claims

1. Process for the preparation of crystalline form I of agomelatine which comprises freeze-drying a solution of agomelatine in an organic solvent selected from C1-C6 alcohols.

2. Process as claimed in claim 1 **characterised in that** said solvent is chosen from tert-butanol and tert-amyl alcohol.

3. Process as claimed in any one of the claims from 1 or 2, **characterised in that** said organic solvent is tert-butanol.

4. Process as claimed in any one of the claims from 1 to 3, **characterised in that** said solution contains 10-100 g of agomelatine per litre of solvent.

5. Process as claimed in claim 4, **characterised in that** said solution contains 40-50 g of agomelatine per litre of solvent.

6. Process as claimed in any one of the claims from 1 to 5, **characterised in that** said solution is frozen to approximately -20°C.

7. Process as claimed in claim 6, **characterised in that** said frozen solution is subjected to a vacuum below or equal to 0.08 mbar.

## Patentansprüche

1. Verfahren zur Herstellung einer kristallinen Form I von Agomelatin umfassend die Gefriertrocknung einer Lösung von Agomelatin in einem organischen Lösungsmittel ausgewählt aus C₁-C₆-Alkoholen.

2. Verfahren gemäß Anspruch 1, **dadurch gekennzeichnet, dass** das Lösungsmittel ausgewählt ist aus tert-Butanol und tert-Amylalkohol.

3. Verfahren gemäß irgendeinem der Ansprüche 1 oder 2, **dadurch gekennzeichnet, dass** das organische Lösungsmittel tert-Butanol ist.

4. Verfahren gemäß irgendeinem der Ansprüche 1 bis 3, **dadurch gekennzeichnet, dass** die Lösung 10 bis 100 g Agomelatin pro Liter Lösungsmittel enthält.

5. Verfahren gemäß Anspruch 4, **dadurch gekennzeichnet, dass** die Lösung 40 bis 50 g Agomelatin pro Liter Lösungsmittel enthält.

6. Verfahren gemäß irgendeinem der Ansprüche 1 bis 5, **dadurch gekennzeichnet, dass** die Lösung auf ungefähr -20°C gefroren wird.

7. Verfahren gemäß Anspruch 6, **dadurch gekennzeichnet, dass** die gefrorene Lösung einem Vakuum von unter oder gleich 0,08 mbar ausgesetzt wird.

## Revendications

1. Procédé de préparation de la forme cristalline I de l'agomélatine qui comprend la lyophilisation d'une solution d'agomélatine dans un solvant organique choisi parmi des alcools en C1-C6.

2. Procédé selon la revendication 1, **caractérisé en ce que** ledit solvant est choisi entre du tert-butanol et de l'alcool tert-amylique.

3. Procédé selon l'une quelconque des revendications 1 ou 2, **caractérisé en ce que** ledit solvant organique est du tert-butanol.

4. Procédé selon l'une quelconque des revendications 1 à 3, **caractérisé en ce que** ladite solution contient 10 à 100 g d'agomélatine par litre de solvant.

5. Procédé selon la revendication 4, **caractérisé en ce que** ladite solution contient 40 à 50 g d'agomélatine par litre de solvant.

6. Procédé selon l'une quelconque des revendications 1 à 5, **caractérisé en ce que** ladite solution est congelée à environ -20°C.

7. Procédé selon la revendication 6, **caractérisé en ce que** ladite solution congelée est soumise à un vide inférieur ou égal à 0,08 mbar.
